# EUROPEAN PATENT SPECIFICATION

(11) **EP 1 912 514 B1**
(45) Date of publication and mention of the grant of the patent: **15.12.2010**
(21) Application number: 06751613.8
(22) Date of filing: 26.04.2006
(51) Int. Cl.: A23D 9/00

(54) **POLYGLYCEROL FATTY ACID ESTER COMPOSITION AND COATING**
POLYGLYCEROLFETTSÄUREESTERPRODUKT UND BESCHICHTUNG
COMPOSITION ET REVETEMENT A BASE D'ESTERS DE POLYGLYCEROLS ET D'ACIDES GRAS

(30) Priority: 26.04.2005 US 114901
(43) Date of publication of application: 23.04.2008
(73) Proprietor: Cargill, Incorporated, Wayzata, MN 55391 (US)
(72) Inventor: LEMKE, Daniel, Cokato, Minnesota 55321 (US)
(74) Representative: Wibbelmann, Jobst
(86) International application number: PCT/US2006/015974
(87) International publication number: WO 2006/116578

(56) References cited:
- US-A- 5 990 230
- US-A- 6 111 055

## Description

### BACKGROUND

In the 1960's, a concerted effort was made to limit the use of various aromatic solvents and some ketones as solvents in coating compositions. As time progressed it became apparent that there were many more compounds that, although harmless themselves, could potentially become transformed into harmful materials by ultraviolet irradiation in the atmosphere.

In the past, a low solvent content was not an important concern for the coatings industry. If better performing vehicles were developed (i.e., those vehicles that required high solvent demand) the industry rapidly switched. The use of higher solids content was a method by which cost and performance could be improved then again, the industry rapidly switched. However, when low volatile organic compound ("VOC ") requirements were imposed on the oil-based alkyd and urethane industries, the problem became much more difficult to solve. Some approaches to solving these and other problems have been explored with alkyds and urethanes.

A simple approach that has been tried is to reduce the amount of solvent used in the coating. While lowering the VOC content, this approach very often produces viscous coatings that may be difficult to apply in a uniform, thin film. Further, the flow characteristics of low solvent coatings can result in reduced penetration of uneven surfaces and certain materials, such as wood.

Another approach which has been utilized is coating without the use of solvents. Powder coating can produce very high quality films. However, the powder coating process must be carried out in a controlled environment, and can often be expensive.

Another approach was to replace regulated solvents with non-regulated, highly volatile solvents such as methyl ethyl ketone or high boiling point mineral spirits. The resultant coatings lacked the regulated VOCs but the highly volatile solvents produced strong odors that were not acceptable to customers. Moreover, the rapid evaporation of the solvent may result in undesirable physical characteristics in the coating, e.g., wrinkling of the film. The use of some highly volatile solvents may also result in a generally softer film.

### SUMMARY

The present application relates to fatty acid based ester materials that can be used as components in coating compositions and/or as intermediates to synthesize coating components. In particular, the application relates to compositions comprising polyglycerol fatty acid esters. The present application describes materials that relate to the environmental concerns surrounding the impact of photochemically reactive solvents that has led to a focused effort by governments to reduce Volatile Organic Compound (VOC) emissions. For example, the polyglycerol fatty acid esters described herein may be useful as a component in a coating composition. In addition to being used as a component in a coating composition, the present polyglycerol fatty acid esters may be employed as an intermediate to form other polymeric materials. In particular, partially esterified polyglycerols may be used as an intermediate in preparing a coating composition component, such as an alkyd or urethane resin.

The present polyglycerol fatty acid esters suitably has a Hydroxyl Value of no more than about 150. The esters are commonly formed from one or more fatty acids and a mixture of polyglycerols, which typically include no more than about 50 wt.% cyclic polyglycerols. The polyglycerol esters may be completely esterified (e.g., have a Hydroxyl Value of no more than about 30) and in certain embodiments have a Hydroxyl Value of no more than about 15. Typically the polyglycerol esters have a fatty acid composition which includes a substantial amount of unsaturated fatty acids, e.g., the polyglycerol fatty acid esters have an Iodine Value of about 80 to 150.

A polyglycerol fatty acid ester may be formed from one or more fatty acid esters which include a substantial amount of unsaturated fatty acid(s). These esters may have an Iodine Value of about 80 to 150. Such polyglycerol esters often have a fatty acid composition which includes about 20 wt.% to 80 wt.% 18:2 fatty acids. The polyglycerol commonly contains no more than about 50% cyclic polyglycerol(s) and, more suitably, no more than about 30% cyclic polyglycerol(s). The polyglycerol fatty acid ester may be a partial ester, which commonly has a Hydroxyl Value of no more than about 150. In other embodiments, the polyglycerol may be essentially completely esterified, e.g., the ester may have a Hydroxyl Value of no more than about 30. The polyglycerol fatty acid ester suitably has a viscosity of no more than about 500 mPa·s (500 cPs) and, very often, about 100 to 250 mPa·s (100 to 250 cPs) at 25°C. Ester materials with viscosities in these ranges can suitably be used to formulate coating compositions with good flow and penetration characteristics.

The present partial polyglycerol fatty acid esters may be used to produce alkyd materials. The alkyds may be formed from a precursor mixture which includes (a) polyglycerol fatty acid ester, e.g., a partial ester having a Hydroxyl Value of about 50 to 150; and (b) dicarboxylic acid, dicarboxylic anhydride or a mixture thereof. The polyglycerol fatty acid ester is desirably formed from a polyglycerol which includes no more than about 30 wt.% cyclic polyglycerols. Suitably the alkyd material may be formed from a polyglycerol ester having an Iodine Value of about 80 to 150. The precursor mixture may also include another fatty acid partial ester, e.g., a fatty acid ester formed from another polyol, such as pentaerythritol. In some instances, a polyol, such as a low molecular weight diol, may be included in the precursor mixture to facilitate the formation of the alkyd. Commonly, the alkyd material is formed from a polyglycerol fatty acid ester, which has a fatty acid composition including about 20 wt.% to 80 wt.% 18:2 fatty acids.

The alkyd materials formed from the present partial polyglycerol fatty acid esters may be used to produce coating compositions. Such coating compositions can include at least about 25 wt.% of the alkyd material and in some instances substantially higher concentrations. These alkyd-based coating compositions suitably have a viscosity of no more than about 5000 mPa·s (5,000 cPs) and, commonly, no more than about 3,000 at 25°C. Alkyd materials formed from the present partial polyglycerol fatty acid esters suitably have a viscosity of no more than about 3000 mPa·s (3,000 cPs) and, typically, about 500 to 2000 mPa·s (500 to 2,000 cPs) at 25°C. Methods of forming substrates with a surface protected with an alkyd-based coating are also provided herein.

A polyurethane material may be formed from a precursor mixture which includes (a) polyglycerol fatty acid ester and (b) polyisocyanate. The polyglycerol fatty acid ester may have a Hydroxyl Value of about 50 to 150. The polyurethane material may be formed from a polyglycerol fatty acid ester which may have an Iodine Value of about 80 to 150. The polyglycerol fatty acid ester is typically formed from polyglycerol which includes no more than about 30 wt.% cyclic polyglycerols. The polyglycerol fatty acid ester may have a fatty acid composition which includes about 20 wt.% to 80 wt.% 18:2 fatty acids. The terms polyurethane, urethane and urethane resin are used interchangeably herein to refer to materials formed from polyol partial esters and polyisocyanate. As used herein, the term "polyisocyanate" refers to a compound which contains two or more isocyanate functional groups. In some embodiments, the polyurethane material is formed from a precursor mixture which includes other polyol partial ester and/or polyol in addition to the polyglycerol fatty acid ester and the polyisocyanate.

The polyurethane materials formed from the present partial polyglycerol fatty acid esters may be used to produce coating compositions. Such coating compositions can include at least about 25 wt.% of the polyurethane material and in some instances substantially higher concentrations. These polyurethane-based coating compositions suitably have a viscosity of no more than about 5000 mPa·s (5,000 cPs) and, more commonly, no more than abut 3,000 at 25°C. Polyurethane materials formed from the present partial polyglycerol fatty acid esters suitably have a viscosity of no more than about 5000 mPa·s (5,000 cPs) and, typically, about 500 to 3000 mPa·s (500 to 3,000 cPs) at 25°C. Methods of forming substrates with a surface protected with an polyurethane-based coating are also provided herein.

### DETAILED DESCRIPTION

Described herein is an esterified polyglycerol material. The esterified polyglycerol material may be a partial and/or complete ester and may used as a coating composition, either by itself or mixed with other components. The polyglycerol is suitably esterified with fatty acids. A fully esterified polyglycerol (e.g., an ester with a Hydroxyl Value of no more than about 30) may be used as a component in a coating composition. A partially esterified polyglycerol (e.g., an ester with a Hydroxyl Value of at least about 50) may be used as an intermediate in the preparation of other coating components, such as alkyds and urethanes. In some embodiments, the partial polyglycerol fatty acid ester may suitably have a Hydroxyl Value of about 50 to 150.

The esters may be produced by reacting polyglycerol reacted with fatty acids, such as soybean fatty acids (i.e., the mixture of fatty acids obtained by the hydrolysis of soybean oil) or other mixtures containing unsaturated fatty acid(s), to produce polyglycerol fatty acid esters. A polyglycerol may have a low content of glycerol and cyclic polyglycerols while having a relatively high content of non-cyclic glycerol oligomers. An exemplary polyglycerol may have an Acid Value of less than 0.1, a Hydroxyl Value of about 750 to 1000 (wet) and 900 to 1200 (dry), a Gardner Color of less than 4 and include about 10 to 25% water. Suitable polyglycerol may have a composition of less than about 5% glycerol, about 60 to 80% non-cyclic glycerol oligomers having two to five glycerol units, and about 10 to 30% cyclic glycerols (determined on a dry basis). One such polyglycerol that is commercially available has an Acid Value of about 0.04, a Hydroxyl Value of 876 (wet) and 1030 (dry), a Gardner Color of 2 and includes 15% water. Such a polyglycerol may also have a composition of about 1% glycerol, 27% diglycerol, 23% triglycerol, 10% tetraglycerol, 20% pentaglycerol, and 19% cyclic glycerols on a dry basis.

Mixtures of fatty acids are typically employed to produce the present polyglycerol esters, which suitably have an Iodine Value of about 80 to 150. The polyglycerol fatty acid ester may be formed from a mixture of fatty acids, which includes about 20 wt.% to 80 wt.% 18:2 fatty acids, and more suitably about 40 wt.% to 60 wt.% 18:2 fatty acids. Commonly, it may be desirable to employ a mixture of fatty acids which includes no more than about 20 wt.% 18:3 fatty acids and, more suitably no more than about 10 wt.% 18:3 fatty acids. One exemplary fatty acid mixture may have a fatty acid composition of about 10 to 15% C16, less than about 10% C18:0, 20 to 30% C18:1, 40 to 60% C18:2, and less than about 10% C18:3. One such fatty acid mixture may have a fatty acid composition of about 12.3% C16, 4.6% C18:0, 24% C18:1, 50.6% C18:2, and 6.0% C18:3.

The ratio of polyglycerol to fatty acid may be varied to produce esters with different Hydroxyl Values. Alternatively, the polyglycerol fatty acid ester may be obtained by transesterification of polyglycerol (or a mixture of polyglycerol and one or more other polyols) with an oil such as soybean oil, linseed oil, or mixtures thereof. Other examples of suitable oils which can be used alone or in combination to produce the present polyglycerol fatty acid esters include cottonseed oil, sunflower oil, corn oil, safflower oil, peanut oil and the like. Examples of other polyols include, for example, diols such as 1,4-dimethylolcyclohexane, 1,4- or 1,3-butanediol, 1,6-hexanediol, neopentylglycol, and 2,2,4-trimethyl-1,3-pentanediol, as well as trimethylolpropane and pentaerythritol.

According to one embodiment, a partial polyglycerol fatty acid ester may be used to make an alkyd product by reaction with a polycarboxylic acid and/or polycarboxylic acid anhydride. The polyglycerol ester may have a Hydroxyl Value of up to about 150. According to an exemplary embodiment, the polyglycerol ester may have a Hydroxyl Value between about 75 and 150. The polyglycerol ester may be part of a precursor mixture which includes dicarboxylic acid, dicarboxylic anhydride or a mixture thereof. The dicarboxylic acids are, for example, aromatic dicarboxylic acids such as phthalic acid, isophthalic acid and terephthalic acid, and/or their anhydrides; cycloaliphatic dicarboxylic acids such as hexahydrophthalic acid, tetrahydrophthalic acid, and endomethylenetetrahydrophthalic acid, and/or their anhydrides; unsaturated aliphatic dicarboxylic acids, such as maleic acid, and/or their anhydrides; and aliphatic dicarboxylic acids, such as succinic acid, glutaric acid, adipic acid, suberic acid, azelaic acid and sebacic acid, and/or their anhydrides. Suitable dicarboxylic acids and dicarboxylic anhydrides may include succinic acid, succinic anhydride, malic acid, tartaric acid, citric acid, diglycolic acid, diglycolic anhydride, glutaric acid, glutaric anhydride, adipic acid, pimelic acid, suberic acid, sebacic acid, fumaric acid, maleic acid, maleic anhydride, itaconic acid phthalic anhydride, isophthalic acid, terephthalic acid, tetrahydrophthalic anhydride, methyltetrahydrophthalic anhydride, endomethylenetetrahydrophthalic anhydride, adipic acid, sebacic acid, HET acid and tetrabromophthalic anhydride. Alkyds formed from adipic acid, maleic acid, phthalic acid, isophthalic acid and/or tetrahydrophthalic acid (and/or the corresponding anhydride(s)) are particularly suitable for use as a coating component.

According to another embodiment, a polyglycerol fatty acid ester may be used to make a urethane product by reacting the polyglycerol ester with a polyisocyanate. The precursor mixture used to form the urethane may include other polyol partial ester and/or polyol in addition to polyglycerol fatty acid partial ester and the polyisocyanate. The polyglycerol ester may have a Hydroxyl Value of up to about 150. According to an exemplary embodiment, the polyglycerol ester may have a Hydroxyl Value of about 50 and 150. Examples of suitable polyisocyanates may include 1,5-naphthylene diisocyanate, 4,4'diphenylmethane diisocyanate, xylylene diisocyanate, tetramethyl xylylene diisocyanate, 4,4'-diphenyl dimethylmethane diisocyanate, di- and tetraalkylene diphenylmethane diisocyanate, 4,4'-dibenzyl diisocyanate, 1,3-phenylene diisocyanate, 1,4-phenylene diisocyanate, the isomers of toluene diisocyanate, 1-methyl-2,4-diisocyanatocyclohexane, 1,6-diisocyanato-2,2,4-trimethyl hexane, 1,6-diisocyanato-2,4,4-trimethyl hexane, 1-isocyanatomethyl-3-isocyanato-1,5,5-trimethyl cyclohexane (IPDI), chlorinated and brominated diisocyanates, phosphorus-containing diisocyanates, 4,4'-diisocyanatophenyl perfluoroethane, tetramethoxybutane-1,4-diisocyanate, butane-1,4-diisocyanate, hexane-1,6-diisocyanate (HDI), dicyclohexylmethane diisocyanate, cyclohexane-1,4-diisocyanate, ethylene diisocyanate, phthalic acid bis-isocyanatoethyl ester; also diisocyanates containing reactive halogen atoms, such as 1-chloromethylphenyl-2,4-diisocyanate, 1-bromomethylphenyl-2,6-diisocyanate or 3,3-bis-chloromethylether-4,4'-diphenyl diisocyanate. Sulphur-containing polyisocyanates are obtained, for example, by reaction of 2 moles of hexamethylene diisocyanate with 1 mole of thiodiglycol or dihydroxydihexyl sulphide. Other examples of diisocyanates are trimethyl hexamethylene diisocyanate, 1,4-diisocyanatobutane, 1,12-diisocyanatododecane and dimeric fatty acid diisocyanate; also tetramethylene diisocyanate, hexamethylene diisocyanate, undecane diisocyanate, dodecamethylene diisocyanate, 2,2,4-trimethylhexane diisocyanate, 1,3-cyclohexane diisocyanate, 1,4-cyclohexane diisocyanate, 1,3- and 1,4-tetramethyl xylene diisocyanate, isophorone diisocyanate, 4,4-dicyclohexanemethane diisocyanate and lysine ester diisocyanate.

In order to regulate viscosity it is possible if desired to add solvents to the coating compositions. Suitable solvents are known paint solvents, such as N-methylpyrrolidone, methoxypropyl acetate, methyl ethyl ketone and/or xylene, for example. If such solvents are employed, they are desirably used in relatively low amounts, e.g., in amounts of no more than about 10 wt.%, preferably no more than about 5 wt.%. Desirably, the coating compositions have a VOC content of no more than about 3 wt.% and, more suitably, no more than about 2 wt.%.

The present coating compositions can be applied in one or more coats to any desired substrates by any desired methods of coating technology, such as spraying, brushing, dipping, flow coating or using rollers and doctor blades. The resulting films can have a dry film thickness of from 0.001 to 0.3 mm. Examples of suitable substrates include metal, plastic, wood or glass. Any of a variety of auxiliaries and additives of coating technology that are known to be used as well, such as pigments, flow-control agents, bubble-preventing additives and/or catalysts, may also be included in the present coating compositions. For example, the present coating compositions may include a drying catalyst, such as a transition metal salt. Examples of suitable drying catalysts include cobalt and zirconium salts, e.g., cobalt carboxylates and/or zirconium carboxylates.

### Examples

The following examples are presented to illustrate the present methods and compositions and to assist one of ordinary skill in making and using the same. The examples are not intended in any way to otherwise limit the scope of the invention.

### Example 1.

1,951g of polyglycerol was placed in a stirred flask with nitrogen sparge. The poly glycerol had an Acid Value of 0.04, a Hydroxyl Value of 876 (wet) and 1030 (dry), a Gardner Color of 2 and included 15% water. The polyglycerol had a composition of 1.2% glycerol, 27.1% diglycerol, 22.6% triglycerol, 10.2% tetraglycerol, 19.9% pentaglycerol, and 19% cyclic glycerols on a dry basis. 7,950g of soybean fatty acid was added, and the mixture was heated to 230°C and held until the acid value was less than two. The nitrogen sparge was switched to nitrogent purge, and the mixture was cooled to 80°C. 3.0g of 85% phosphoric acid was added to partially neutralize the mixture, and the mixture was stirred for an additional 30 minutes. 5.0g of calcium oxide and 20g of filter aid were added and the mixture was stirred for an hour at 80°C. The product was filtered at 80°C using a bed of filter aid in a Buchner vacuum funnel.

The product was analyzed and found to have an Acid Value of 1.2, a Hydroxyl Value of 17.7, a Gardner Color of 1, a viscosity of 168 mPa·s (168cPs) at 25°C, and a density of 0.946g/mL. The properties of the product and the film made from it are reproduced as sample 1 in Table 1.

The product was used to make a 3 mil film using a draw down bar. The film formed in 1.4 hours and was thoroughly dry in 2.1 hours. The film was about 5% wrinkled.

### Examples 2-4.

Three more samples (numbered 2-4 in Table 1) were made varying the polyglycerol to soybean fatty acid ester ratio. The properties (Hydroxyl Value (OH), Acid Value (AV), Viscosity in mPa·s (cP), Gardner Color, Film Formation Time, Film Dry Time, and % Wrinkle) of the resulting products are reproduced in Table 1. Times are in hours. Times in parentheses are for 3 mil films. Times not in parentheses are for 0.75 mil films.

**Table 1**

| **No.** | **OH** | **AV** | **Viscosity at 258C** | **Gardner Color** | **Film Formation Time*** | **Film Dry Time*** | **% Wrinkle** |
|---|---|---|---|---|---|---|---|
| 1 | 17.7 | 1.2 | 168 | 1+ | 1.4 | 2.1 | 5 |
| 2 | 18.0 | 2.0 | 168 | 1+ | 1.4 (2.6) | 5.2 (3.1) | 5 |
| 3 | 13.4 | 1.7 | 160 | 2 | 2.4 (2.8) | 3.2 (3.5) | 5 |
| 4 | 7.2 | 0.7 | 179 | 1+ | 3.4 (2.9) | 7.2 (3.5) | 5 |

### Example 5.

A blend was prepared at room temperature by combining pentaerythritol fatty acid ester made with soybean fatty acid, with the polyglycerol fatty acid ester of Example 1 in a weight ratio of 60% pentaerithritol fatty acid ester to 40% polyglycerol fatty acid ester. The product was used to make a 3 mil film using a draw down bar. The film formed in 2.8 hours and was thoroughly dry in 4.2 hours. The film was about 5% wrinkled.

### Example 6.

Into a 2L four neck round bottomed flask equipped with a mechanical stirrer, nitrogen spare, short path vertical condenser and electric heat was placed 235.9g of polyglycerol and 961.2g of linseed fatty acid. Nitrogen sparge and agitation were set on medium. The mixture was heated to 230°C. At 230°C, the mixture was held until the Acid Value was less than two. Once the Acid Value was less than two the nitrogen sparge was switched to nitrogen purge. The mixture was cooled to 80°C. At 80°C, the temperature was maintained and 0.36g of 85% phosphoric acid was added to partially neutralize the mixture. The mixture was stirred for thirty minutes. After thirty minutes 0.6 g calcium oxide and 2.5 g filter aid were added to the mixture and stirred for one hour at 80°C.

The product was filtered at 80°C through a bed of filter aid using a Buchner vacuum filter. The final product was analyzed and found to have an Acid Value of 0.2, a Hydroxyl Value of 23, a Gardner Color of 9, and a viscosity of 260 mPa·s (260 cPs) at 25°C. The product was used to make a 3 mil film using a draw down bar. The film formed in 1.6 hours and was thoroughly dry in 3.4 hours. The film was about 60% wrinkled.

### Example 7.

Into a 2L four neck round bottomed flask equipped with a mechanical stirrer, nitrogen spare, short path vertical condenser and electric heat was placed 77.2g of pentaerythritol, 561.3g of linseed oil, and 0.32g of calcium oxide. Nitrogen sparge and agitation were set on medium. The mixture was heated to 250°C. At 250°C, the mixture was held for one hour after it cleared, total of two hours. The mixture was cooled to 80°C. At 80°C, 69.2g of polyglycerol and 507g of soybean fatty acid were added and the mixture heated to 230°C. At 230°C the mixture was held until the Acid Value was less than 2. The mixture was cooled to less than 80°C and analyzed to have an Acid Value of 1.1, a Hydroxyl Value of 73, and the mixture was clear.

### Example 8.

The product of example 7 was split into two fractions. 243g of soybean fatty acid was added to the first fraction of 575 g. The second fraction was reserved for Example 9. The reagents were placed into a two liter round bottom flask equipped with a mechanical stirrer, nitrogen spare, short path vertical condenser and electric heat. The mixture was heated to 230°C and held until the Acid Value was two or less. The reaction was cooled to less than 80°C when the Acid Value was 2.5. At 80°C 0.16g of 85% phosphoric acid was added and mixed for one hour. 0.6g of calcium oxide and 6g of filter aid were added. The mixture was mixed for one hour and then filtered through a bed of filter aid using a Buchner filter funnel. The temperature was maintained and 0.36g of 85% phosphoric acid was added to partially neutralize the mixture. The mixture was stirred for thirty minutes. After thirty minutes 0.6g of calcium oxide and 2.5g of filter aid were added to the mixture and stirred for one hour at 80°C.

The product was filtered at 80°C through a bed of filter aid using a Buchner vacuum filter. The final product was analyzed and found to have an Acid Value of 2.5, a Hydroxyl Value of 12.9, a Gardner Color of 5+, a viscosity of 160 mPa·s (160 cPs) at 25°C, and a density of 0.932 g/mL. The product was used to make a 3 mil film using a draw down bar. The film formed in 2.2 hours and was thoroughly dry in 3.8 hours. The film was about 30% wrinkled.

### Example 9.

300 g of the second fraction of reserved from Example 7 was combined with 32g of phthalic anhydride and 1.33g of maleic anhydride. The reagents were placed into a one liter round bottom flask equipped with a mechanical stirrer, nitrogen purge, short path vertical condenser and electric heat. The mixture was heated to 170°C and held for three hours. The mixture was ramped to 215°C over four hours and then held till the Acid Value was five or less. The reaction was cooled to less than 80°C when the Acid Value was 5.2.

At 80°C filter aid was added, mixed for one hour and then filtered using a Buchner filter funnel. The final product was analyzed and found to have an Acid Value of 5.2, a Hydroxyl Value of 19, a Gardner Color of 9+, and a viscosity of 515 mPa·s (515 cPs) at 25°C. The product was used to make a 3 mil film using a draw down bar. The film formed in 0.8 hours and was thoroughly dry in 3.8 hours. The film was about 5% wrinkled.

### Example 10.

Five polyglycerol esters were made using different weight ratios of polyglycerol to linseed oil. For each of the five polyglycerol esters, linseed oil, glycerin and polyglycerol were combined in a reaction flask. A nitrogen sparge was applied. The mixture was heated to 230°C. The temperature was maintained at 230°C until the Acid Value was less than 2. Once the Acid Value was less than 2, the batch was cooled and filtered.

Each of the five polyglycerol esters was then used to make an alkyd. The polyglycerol ester was placed into a two liter four neck round bottomed flask equipped with a mechanical stirrer, nitrogen purge, Dean Stark trap and vertical condenser and electric heat. Phthalic anhydride, maleic anhydride, and xylene were added. The mixture was ramped from 170°C to 210°C over a four hour period. The Dean Stark trap was removed at this point and the xylene was collected. The mixture was heated to 230°C and maintained at that temperature until xylene ceased to distill (about two hours). The product was cooled and filtered. Table 2 shows the viscosity at 25°C, Hydroxyl Value, and Acid Value for each of the five polyglycerols, and the alkyd made from each.

**Table 2**

| Reference | Starting Polyglycerol Ester | | | Final Alkyd | | |
|---|---|---|---|---|---|---|
| | Viscosity (cP) | OH | AV | Viscosity (cP) | OH | AV |
| 1 | --- | 48 | 1.1 | 536 | 16 | 1.6 |
| 2 | 312 | 77 | 0.6 | 918 | 21 | 1.9 |
| 3 | 354 | 88 | 0.3 | 1679 | 22 | 2.0 |
| 4 | 423 | 102 | 0.05 | 2166 | 20 | 0.7 |
| 5 | 495 | 116 | 0.04 | 5451 | 21 | 5.5 |

### Example 11.

7135g of soybean fatty acid, 943g of pentaerythritol (10% dipentaerythritol) 853g of polyglycerol (Hydroxyl Value = 890) was placed in a twelve liter round bottom flask equipped with a mechanical stirrer, nitrogen purge, short path vertical condenser and electric heat. The mixture was heated to 210°C and held until the Acid Value was less than one. Analysis showed the product to have an Acid Value of 0.3, a Hydroxyl Value of 102, and a Gardner color of 2. At this point the reaction mixture was cooled to less than 120°C.

68.4g of ethylene glycol was added and mixed for fifteen minutes. Analysis showed the product to have an Acid Value of 0.2 and a Hydroxyl Value of 113. The nitrogen sparge was switched to nitrogen purge. 1174g of phthalic anhydride and 47.6g of maleic anhydride was added. The mixture was ramped to 210°C over eight hours. Analysis showed the mixture had an Acid Value of 12 and a Hydroxyl Value of 27. The mixture was heated to 250°C and then held until the Acid Value was less than ten. The mixture was cooled to less than 80°C.

Filter aid and 5g of calcium oxide were added. The mixture was mixed for one hour and then filtered using a Buchner filter funnel. The final product was analyzed and found to have an Acid Value of 7.3, a Hydroxyl Value of 20, a Gardner Color of 4+, and a Viscosity of 1350 mPa·s (1350 cPs) at 25°C. The product was used to make a 3 mil film using a draw down bar. The film formed in 2.6 hours and was thoroughly dry in 3.1 hours. The film was about 5% wrinkled and had a hardness of about 4B.

### Example 12.

Polyglycerol esters produced according to Example 7 or 10 (300 g) may be combined with diisocyanate, e.g., 36g of hexamethylene diisocyanate. The reagents may be placed into a one liter round bottom flask equipped with a mechanical stirrer, nitrogen purge, short path vertical condenser and electric heat. The mixture may be heated to 170°C and held for three hours. The mixture then may be ramped to 215°C over four hours and then held until the Hydroxyl Value lowers to 30 or less. The reaction may be cooled to less than 80°C at this point.

Once the reaction mixture has cooled to 80°C, filter aid may be added, mixed for one hour and then filtered using a Buchner filter funnel.

### Example 13.

Test solutions were prepared by blending at room temperature 17.58g of a product obtained from one of Examples 1-8 and 10, 0.218g of 12% cobalt carboxylate, 0.072g of 12% zirconium carboxylate, and 2.13g of mineral spirits. The blends were placed on a shaking table and mixed for one hour. A 1.5 micron film was obtained by using a 3 micron draw down bar. The film was cast on an 8" by 8" clean glass plate. A circular drier was used to obtain dry time data. Pencil hardness was used to determine film hardness. Table 3 shows the film forming time, thorough drying time, % wrinkle, 1 week and 2 week hardness for these films.

**Table 3**

| Example | Film Formed | Through Dry | % Wrinkle | Hardness 1 wk | Hardness 2 wk |
|---|---|---|---|---|---|
| 1 | 1.8, 1.4 | 2.1,2.1 | 5 | 6B | 6B |
| 2 | 1.4, 2.6 | 5.2, 3.0 | 5 | 6B | 6B |
| | (2.6) | (3.1) | | | |
| 3 | 2.4 | 3.2 | 5 | 6B | 6B |
| | (2.8) | (3.5) | | | |
| 4 | 3.4 | 7.2 | 5 | 6B | 6B |
| | (2.9) | (3.5) | | | |
| 5 | 2.8 | 4.2 | 5 | 6B | 6B |
| 6 | 1.6 | 3.4 | 60 | | 2B |
| 7 | 2.2 | 3.8 | 30 | 6B | 6B |
| 8 | 0.8, 1.2 | 3.8, 3.4 | 5 | 5B | 5B |
| 11 | 2.6/1.4 | 3.2/3.4 | 10/5 | 4B | 4B |

### Illustrative Embodiments

A number of illustrative embodiments of the present methods and compositions are described below. The embodiments described are intended to provide illustrative examples of the present methods and compositions and are not intended to limit the scope of the invention.

According to one embodiment, a polyglycerol fatty acid ester may have a Hydroxyl Value of no more than about 150; a viscosity of about 100 to 250 cPs at 25°C; an Iodine Value of about 80 to 150. The polyglycerol may include no more than about 30 wt.% cyclic polyglycerols; and the ester has a fatty acid composition which includes about 20 wt.% to 80 wt.% 18:2 fatty acids.

Another embodiment relates to a method for preparing a coated surface comprising: applying a coating composition comprising a polyglycerol fatty acid ester to a surface; and curing the coating composition.

The polyglycerol fatty acid ester may alternatively have a Hydroxyl Value of no more than about 30. Alternatively, the polyglycerol fatty acid ester may have a Hydroxyl Value of no more than about 25. Alternatively, the polyglycerol fatty acid ester may have a Hydroxyl Value of no more than about 15.

The polyglycerol fatty acid ester may have a Hydroxyl Value of about 50 to 150.

The polyglycerol fatty acid ester may have a fatty acid composition which includes at least about 80 wt.% 2 fatty acids having 16 to 18 carbon atoms.

The polyglycerol fatty acid ester may have an Acid Value of no more than about 5.

The polyglycerol fatty acid ester may include a VOC content of no more than about 3 wt.% and, more suitably, no more than about 2 wt.%.

According to another embodiment, an alkyd material may be formed from a precursor mixture which includes a polyglycerol fatty acid ester having a Hydroxyl Value of about 50 to 150; and a dicarboxylic acid, dicarboxylic anhydride or a mixture thereof. The polyglycerol ester may be formed from a polyglycerol which includes no more than about 30 wt.% cyclic polyglycerols. The polyglycerol ester may have an Iodine Value of about 80 to 150 and a fatty acid composition which includes about 20 wt.% to 80 wt.% 18:2 fatty acids.

An alkyd material may be formed from a precursor mixture which further comprises polyol wherein the polyol is not a polyglyceride. The polyol may be a diol having a molecular weight of less than about 150 and, more desirably less than 100. Examples of such a diols include, for example, diols such as ethyleneglycol, 1,2-propanediol, 1,3- propanediol, 1,4- butanediol or 1,3-butanediol, 1,6-hexanediol, neopentylglycol, and 1,3-pentanediol.

The precursor mixture may also comprise another polyol fatty acid ester, such as pentaerythritol fatty acid ester. The pentaerythritol fatty acid ester may have a Hydroxyl Value of about 50 to 125 and an Iodine Value of about 80 to 150.

The dicarboxylic acid anhydride used to form the present polyurethane may include phthalic anhydride, maleic anhydride or a mixture thereof. The dicarboxylic acid may include terephthalic acid, isophthalic acid or a mixture thereof.

The coating composition may comprise at least about 25 wt.% of the alkyd. In some embodiments, the coating composition may comprise about 60 wt.% or more of the alkyd.

The alkyd-based coating composition may have a VOC content of no more than about 3 wt.% and, more suitably, no more than about 2 wt.%.

Yet another embodiment relates to a method for preparing a coated surface comprising: applying the coating composition comprising an alkyd formed from a polyglycerol fatty acid ester to a surface; and curing the coating composition.

According to another embodiment, a polyurethane material may be formed from a precursor mixture which includes (a) polyglycerol fatty acid ester and (b) polyisocyanate. The polyglycerol fatty acid ester may have a Hydroxyl Value of about 50 to 150 and an Iodine Value of about 80 to 150; The polyglycerol fatty acid ester may be formed from polyglycerol which includes no more than about 25 wt.% cyclic polyglycerols. The polyglycerol fatty acid ester may have a fatty acid composition which includes about 20 wt.% to 80 wt.% 18:2 fatty acids.

Another embodiment relates to a method for preparing a coated surface comprising: applying a coating composition comprising a polyurethane formed from a polyglycerol fatty acid ester to a surface; and curing the coating composition.

The polyurethane material may have a viscosity of no more than 5000 mPa·s (5,000 cPs) at 25°C. More suitably, the polyurethane may have a viscosity of about 500 to 5000 mPa·s (500 to 5000 cPs) at 25°C.

A coating composition comprising a polyurethane formed from a polyglycerol fatty acid ester may comprise at least about 25 wt.% of the polyurethane.

A coating composition comprising a polyurethane formed from a polyglycerol fatty acid ester may contain a VOC content of no more than about 3 wt.% and, more desirably, no more than about 2 wt.%.

The polyurethane may be formed from a precursor mixture which further comprises a polyol fatty acid ester having a Hydroxyl Value of about 75 to 125 and an Iodine Value of about 80 to 150, wherein the polyol is not polyglycerol.

According to another embodiment, a coating composition may comprise an alkyd component formed from a reaction mixture comprising a polyglycerol fatty acid ester having a Hydroxyl Value of about 75 to about 150, and a diacid reagent selected from the group consisnting of a dicarboxylic acid, a dicarboxylic anhydride, and mixtures thereof. The polyglycerol may include no more than about 25 wt.% cyclic polyglycerols.

According to another embodiment, a coating composition may comprise an alkyd component formed from a reaction mixture comprising a polyglycerol fatty acid ester having a Hydroxyl Value of about 75 to about 150, and a diacid reagent selected from the group consisnting of a dicarboxylic acid, a dicarboxylic anhydride, and mixtures thereof. The coating composition may have a viscosity of about 100 cPs to about 5000 cPs at 25°C, and a VOC content of no more than about 2 wt.%.

According to yet another embodiment, a coating composition may comprise a urethane component formed from a reaction mixture comprising a polyglycerol fatty acid ester having a Hydroxyl Value of about 75 to about 150, and a polyisocyanate. The polyglycerol includes no more than about 25 wt.% cyclic polyglycerols.

According to another embodiment, a coating composition may comprise a urethane component formed from a reaction mixture comprising a polyglycerol fatty acid ester having a Hydroxyl Value of about 75 to about 150 and a polyisocyanate. The coating composition may have a viscosity of about 100 to 5000 mPa·s (100 cPs to about 5000 cPs) at 25°C, and a VOC content of no more than about 2 wt.%.

According to another embodiment, a polyol fatty acid ester composition may have a Hydroxyl Value of no more than about 20, an Iodine Value of about 80 to 150. The polyol may comprise a mixture of polyglycerol and pentaerythritol.

According to another embodiment, a polyglycerol fatty acid ester composition may have a viscosity of about 100 to 5000 mPa·s (100 cPs to 5000 cPs) at 25°C, a Hydroxyl Value of no more than about 20, an Iodine Value of about 80 to 150, and a VOC content of no more than about 2 wt.%.

According to yet another embodiment, a polyglycerol fatty acid ester composition may have a viscosity of about 100 to 5000 mPa·s (100 cPs to 5000 cPs) at 25°C, a Hydroxyl Value of about 75 to 150, an Iodine Value of about 80 to 150, and a VOC content of no more than about 2 wt.%.

According to another embodiment, a polyglycerol soybean fatty acid ester composition may have a Hydroxyl Value of no more than about 20, a viscosity of about 100 to 5000 mPa·s (100 cPs to 5000 cPs) at 25°C, and a VOC content of no more than about 2 wt.%.

According to another embodiment, a polyglycerol soybean fatty acid ester composition may have a Hydroxyl Value of about 75 to 150, a viscosity of about 100 to 5000 mPa·s (100 to 5000 cPs) at 25°C, and a VOC content of no more than about 2 wt.%.

## Claims

1. A polyglycerol fatty acid ester having a Hydroxyl Value no more than about 150; a viscosity of about 100 to 250 mPa·s (cPs) at 25°C; and an Iodine Value of about 80 to 150; wherein the polyglycerol fatty acid ester is formed from polyglycerol which includes no more than about 30 wt. % cyclic polyglycerols; and the ester has a fatty acid composition which includes about 20 wt. % to 80 wt. % 18:2 fatty acids.

2. The polyglycerol fatty acid ester of claim 1 having a Hydroxyl Value of about 50 to 150.

3. The polyglycerol fatty acid ester of claim 1 having a Hydroxyl Value of no more than about 30.

4. The polyglycerol fatty acid ester of claim 1 having a fatty acid composition which includes no more than about 10 wt. % 18:3 fatty acids.

5. The polyglycerol fatty acid ester of claim 1 having a fatty acid composition which includes at least about 80 wt. % fatty acids having 16 to 18 carbon atoms.

6. The polyglycerol fatty acid ester of claim 1 wherein said polyglycerol ester has an Acid Value of no more than about 5.

7. The polyglycerol fatty acid ester of claim 1 wherein said polyglycerol ester has Volatile Organic Compound (VOC) content of no more than about 3 wt. %.

8. An alkyd material formed from a precursor mixture which includes (a) polyglycerol fatty acid ester having a Hydroxyl Value of about 50 to 150; and (b) dicarboxylic acid, dicarboxylic anhydride or a mixture thereof;
wherein the polyglycerol ester has an Iodine Value of about 80 to 150 and a fatty acid composition, which includes about 20 wt. % to 80 wt. % 18:2 fatty acids; and the polyglycerol fatty acid ester is formed from a polyglycerol which includes no more than about 30 wt. % cyclic polyglycerols.

9. The alkyd material of claim 8 wherein the precursor mixture further comprises polyol partial ester; wherein the polyol is not glycerin or a polyglycerol.

10. The alkyd material of claim 8 wherein the precursor mixture further comprises diol having a molecular weight of less than 100.

11. The alkyd material of claim 8 wherein the precursor mixture further comprises pentaerythritol fatty acid ester.

12. The alkyd material of claim 8 wherein said material has a VOC content of no more than about 3 wt. %.

13. The alkyd material of claim 8 wherein dicarboxylic anhydride comprises phthalic acid, isophthalic acid, terephthalic acid, maleic acid or a mixture thereof.

14. A coating composition comprising at least about 25 wt. % of the alkyd material of claim 8.

15. A method for preparing a coated surface, the method comprising:
applying a coating composition comprising the alkyd material of claim 8 to a surface; and
curing the coating composition.

16. A polyurethane material formed from a precursor mixture which includes (a) polyglycerol fatty acid ester having an Hydroxyl Value of about 50 to 150 and an Iodine Value of about 80 to 150; and (b) polyisocyanate;
wherein the polyglycerol fatty acid ester has a fatty acid composition which includes about 20 wt. % to 80 wt. % 18:2 fatty acids; and the polygycerol fatty acid ester is formed from a polyglycerol which includes no more than about 30 wt. % cyclic polyglycerols.

17. The polyurethane material of claim 16 wherein the polyglycerol fatty acid ester has a viscosity of about 100 to 500 mPa·s (cPs) at 25°C.;

18. The polyurethane material of claim 16 wherein the polyurethane has a viscosity of about 500 to 5000 mPa·s (cPs) at 25°C.

19. The polyurethane material of claim 16 wherein the polyisocyanate includes toluenediisocyanate, diphenylmethylenediisocyanate, hexamethylene diisocyanate or a mixture thereof.

20. The polyurethane material of claim 16 wherein the precursor mixture further comprises polyol, polyol fatty acid ester or a mixture thereof; wherein the polyol is not polyglycerol.

## Patentansprüche

1. Polyglycerolfettsäureester mit einer Hydroxylzahl von nicht mehr als etwa 150; einer Viskosität von etwa 100 bis 250 mPa·s (cPs) bei 25 °C; und einer Iodzahl von etwa 80 bis 150; wobei der Polyglycerolfettsäureester aus Polyglycerol gebildet ist, das nicht mehr als etwa 30 Gew.-% cyclische Polyglycerole einschließt, und der Ester eine Fettsäure-Zusammensetzung aufweist, die etwa 20 Gew.-% bis 80 Gew.-% 18:2-Fettsäuren einschließt.

2. Polyglycerolfettsäureester nach Anspruch 1 mit einer Hydroxylzahl von etwa 50 bis 150.

3. Polyglycerolfettsäureester nach Anspruch 1 mit einer Hydroxylzahl von nicht mehr als etwa 30.

4. Polyglycerolfettsäureester nach Anspruch 1 mit einer Fettsäure-Zusammensetzung, die nicht mehr als etwa 10 Gew.-% 18:3-Fettsäuren einschließt.

5. Polyglycerolfettsäureester nach Anspruch 1 mit einer Fettsäure-Zusammensetzung, die mindestens etwa 80 Gew.-% Fettsäuren mit 16 bis 18 Kohlenstoffatomen einschließt.

6. Polyglycerolfettsäureester nach Anspruch 1, bei dem der Polyglycerolester eine Säurezahl von nicht mehr als etwa 5 aufweist.

7. Polyglycerolfettsäureester nach Anspruch 1, bei dem der Polyglycerolester einen Gehalt an flüchtigen organischen Verbindungen (VOC) von nicht mehr als etwa 3 Gew.-% aufweist.

8. Alkydmaterial, gebildet aus einer Vorstufen-Mischung, die (a) Polyglycerolfettsäureester mit einer Hydroxylzahl von etwa 50 bis 150; und (b) Dicarbonsäure, Dicarbonsäureanhydrid oder eine Mischung derselben einschließt;
wobei der Polyglycerolester eine Iodzahl von etwa 80 bis 150 und eine Fettsäure-Zusammensetzung aufweist, die etwa 20 Gew.-% bis 80 Gew.-% 18:2-Fettsäuren einschließt; und der Polyglycerolfettsäureester aus einem Polyglycerol gebildet ist, das nicht mehr als etwa 30 Gew.-% cyclische Polyglycerole einschließt.

9. Alkydmaterial nach Anspruch 8, bei dem die Vorstufen-Mischung weiter Polyolpartialester umfasst, wobei das Polyol nicht Glycerin oder ein Polyglycerol ist.

10. Alkydmaterial nach Anspruch 8, bei dem die Vorstufen-Mischung weiter Diol mit einem Molekulargewicht von weniger als 100 umfasst.

11. Alkydmaterial nach Anspruch 8, bei dem die Vorstufen-Mischung weiter Pentaerythritfettsäureester umfasst.

12. Alkydmaterial nach Anspruch 8, bei dem das Material einen Gehalt an VOC von nicht mehr als etwa 3 Gew.-% aufweist.

13. Alkydmaterial nach Anspruch 8, bei dem das Dicarbonsäureanhydrid Phthalsäure, Isophthalsäure, Terephthalsäure, Maleinsäure oder eine Mischung derselben umfasst.

14. Beschichtungsmittelzusammensetzung, umfassend mindestens etwa 25 Gew.% des Alkydmaterials nach Anspruch 8.

15. Verfahren zur Herstellung einer beschichteten Obertläche, wobei das Verfahren umfasst:
Auftragen einer Beschichtungsmittelzusammensetzung, die das Alkydmaterial nach Anspruch 8 umfasst, auf eine Oberfläche, und Härten der Beschichtungsmittelzusammensetzung.

16. Polyurethanmaterial, gebildet aus einer Vorstufen-Mischung, die einschließt: (a) Polyglycerolfettsäureester mit einer Hydroxylzahl von etwa 50 bis 150 und einer Iodzahl von etwa 80 bis 150; und (b) Polyisocyanat;
wobei der Polyglycerolfettsäureester eine Fettsäure-Zusammensetzung aufweist, die etwa 20 Gew.-% bis 80 Gew.-% 18:2-Fettsäuren einschließt; und der Polyglycerolfettsäureester aus einem Polyglycerol gebildet ist, das nicht mehr als etwa 30 Gew.-% cyclische Polyglycerole einschließt.

17. Polyurethanmaterial nach Anspruch 16, bei dem der Polyglycerolfettsäureester eine Viskosität von etwa 100 bis 500 mPa·s (cPs) bei 25 °C aufweist.

18. Polyurethanmaterial nach Anspruch 16, bei dem das Polyurethan eine Viskosität von etwa 500 bis 5000 mPa·s (cPs) bei 25 °C aufweist.

19. Polyurethanmaterial nach Anspruch 16, bei dem das Polyisocyanat Toluoldiisocyanat, Diphenylmethylendiisocyanat, Hexamethylendiisocyanat oder eine Mischung derselben einschließt.

20. Polyurethanmaterial nach Anspruch 16, bei dem die Vorstufen-Mischung weiter Polyol, Polyolfettsäureester oder eine Mischung derselben umfasst, wobei das Polyol nicht Polyglycerol ist.

## Revendications

1. Ester d'acide gras de polyglycérol ayant un indice d'hydroxyle de pas plus d'environ 150 ; une viscosité d'environ 100 à 250 mPa·s (cPs) à 25 °C ; et un indice d'iode d'environ 80 à 150 ; dans lequel l'ester d'acide gras de polyglycérol est formé à partir d'un polyglycérol qui n'inclut pas plus d'environ 30 % en poids de polyglycérols cycliques ; et l'ester a une composition d'acide gras qui inclut environ 20 % en poids à 80 % en poids d'acides gras 18:2.

2. Ester d'acide gras de polyglycérol selon la revendication 1 ayant un indice d'hydroxyle d'environ 50 à 150.

3. Ester d'acide gras de polyglycérol selon la revendication 1 ayant un indice d'hydroxyle de pas plus d'environ 30.

4. Ester d'acide gras de polyglycérol selon la revendication 1 ayant une composition d'acide gras qui n'inclut pas plus d'environ 10 % en poids d'acides gras 18:3.

5. Ester d'acide gras de polyglycérol selon la revendication 1 ayant une composition d'acide gras qui inclut au moins environ 80 % en poids d'acides gras ayant 16 à 18 atomes de carbone.

6. Ester d'acide gras de polyglycérol selon la revendication 1, dans lequel ledit ester de polyglycérol a un indice d'acide de pas plus d'environ 5.

7. Ester d'acide gras de polyglycérol selon la revendication 1, dans lequel ledit ester de polyglycérol a une teneur en composés organiques volatiles (COV) de pas plus d'environ 3 % en poids.

8. Matériau d'alkyde formé à partir d'un mélange précurseur qui inclut (a) un ester d'acide gras de polyglycérol ayant un indice d'hydroxyle d'environ 50 à 150 ; et (b) un acide dicarboxylique, un anhydride dicarboxylique ou un mélange de ceux-ci ; dans lequel l'ester de polyglycérol a un indice d'iode d'environ 80 à 150 et une composition d'acide gras qui inclut environ 20 % en poids à 80 % en poids d'acides gras 18:2 ; et l'ester d'acide gras de polyglycérol est formé à partir d'un polyglycérol qui n'inclut pas plus d'environ 30 % en poids de polyglycérols cycliques.

9. Matériau d'alkyde selon la revendication 8, dans lequel le mélange précurseur comprend en outre un ester partiel de polyol ; dans lequel le polyol n'est pas la glycérine, ni un polyglycérol.

10. Matériau d'alkyde selon la revendication 8, dans lequel le mélange précurseur comprend en outre un diol ayant un poids moléculaire de moins de 100.

11. Matériau d'alkyde selon la revendication 8, dans lequel le mélange précurseur comprend en outre un ester d'acide gras de pentaérythritol.

12. Matériau d'alkyde selon la revendication 8, dans lequel ledit matériau a une teneur en COV de pas plus d'environ 3 % en poids.

13. Matériau d'alkyde selon la revendication 8, dans lequel l'anhydride dicarboxylique comprend l'acide phtalique, l'acide isophtalique, l'acide téréphtalique, l'acide maléique ou un mélange de ceux-ci.

14. Composition de revêtement comprenant au moins environ 25 % en poids du matériau d'alkyde selon la revendication 8.

15. Procédé de préparation d'une surface revêtue, le procédé comprenant :
l'application d'une composition de revêtement comprenant le matériau d'alkyde selon la revendication 8 à une surface ; et
le durcissement de la composition de revêtement.

16. Matériau de polyuréthane formé à partir d'un mélange précurseur qui inclut (a) un ester d'acide gras de polyglycérol ayant un indice d'hydroxyle d'environ 50 à 150 et un indice d'iode d'environ 80 à 150 ; et (b) un polyisocyanate ;
dans lequel l'ester d'acide gras de polyglycérol a une composition d'acide gras qui inclut environ 20 % en poids à 80 % en poids d'acides gras 18:2 ; et l'ester d'acide gras de polyglycérol est formé à partir d'un polyglycérol qui n'inclut pas plus d'environ 30 % en poids de polyglycérols cycliques.

17. Matériau de polyuréthane selon la revendication 16, dans lequel l'ester d'acide gras de polyglycérol a une viscosité d'environ 100 à 500 mPa·s (cPs) à 25°C.

18. Matériau de polyuréthane selon la revendication 16, dans lequel le polyuréthane a une viscosité d'environ 500 à 5 000 mPa·s (cPs) à 25 °C.

19. Matériau de polyuréthane selon la revendication 16, dans lequel le polyisocyanate inclut le diisocyanate de toluène, le diisocyanate de diphénylméthylène, le diisocyanate d'hexaméthylène ou un mélange de ceux-ci.

20. Matériau de polyuréthane selon la revendication 16, dans lequel le mélange précurseur comprend en outre un polyol, un ester d'acide gras de polyol ou un mélange de ceux-ci ; dans lequel le polyol n'est pas un polyglycérol.
